# EUROPÄISCHE PATENTSCHRIFT

(11) **EP 2 389 212 B1**
(45) Veröffentlichungstag und Bekanntmachung des Hinweises auf die Patenterteilung: **22.11.2017**
(21) Anmeldenummer: 10702819.3
(22) Anmeldetag: 22.01.2010
(51) Int. Cl.: A61M 5/31, A61M 5/34

(54) **SPRITZE**
SYRINGE
SERINGUE

(30) Priorität: 26.01.2009 DE 102009007250
(43) Veröffentlichungstag der Anmeldung: 30.11.2011
(73) Patentinhaber: Arzneimittel GmbH Apotheker Vetter & Co. Ravensburg, 88212 Ravensburg (DE)
(72) Erfinder: PUPKE, Holger, 88376 Koenigseggwald (DE); ROEDLE, Tilman, 88364 Wolfegg (DE)
(74) Vertreter: Gleiss, Alf-Olav
(86) Internationale Anmeldenummer: PCT/EP2010/000357
(87) Internationale Veröffentlichungsnummer: WO 2010/084006

(56) Entgegenhaltungen:
- EP-A2- 1 779 882
- WO-A1-02/055140
- WO-A1-2004/037335
- WO-A1-2009/144583
- JP-A- 2003 024 441
- US-A- 4 676 530
- US-A- 5 462 531
- US-A- 5 858 000

## Beschreibung

Die Erfindung betrifft eine Spritze gemäß Oberbegriff des Anspruchs 1.

Spritzen der hier angesprochenen Art sind bekannt. Sie weisen einen Spritzenzylinder und ein sich an diesen anschließendes distales Ende auf, das als Spritzenkonus ausgebildet ist. Das distale Ende umfasst einen in radialer Richtung zurückspringenden Bereich, wobei - in axialer Richtung gesehen - eine Kante gebildet wird, die sich in Umfangsrichtung erstreckt. Insbesondere wenn ein Spritzenkörper der Spritze aus Glas besteht, ist es aus fertigungstechnischen Gründen beziehungsweise aufgrund der im Material bestehenden Spannungen nicht möglich, diese Kante mit einem spitzen Winkel beziehungsweise rechtwinklig auszubilden. Sie weist daher eine Fase auf und/oder hat - im Längsschnitt gesehen - die Form einer Rampe. Die Spritze weist ein Aufsatzstück auf, das einen Klemmbereich umfasst. Dieser hat vorzugsweise einen Innendurchmesser, der bei von dem Spritzenkörper getrenntem Aufsatzstück kleiner ist als der Außendurchmesser des in radialer Richtung zurückspringenden Bereichs am distalen Ende der Spritze. Wird das Aufsatzstück auf die Spritze aufgesetzt, sodass der Klemmbereich mit dem radial zurückspringenden Bereich zusammenwirkt, ergibt sich eine Aufweitung des Klemmbereichs in radialer Richtung, sodass hier Haltekräfte in den in radialer Richtung zurückspringenden Bereich der Spritze eingeleitet werden. Der Klemmbereich weist eine distale Kante auf.

Insgesamt wird das Aufsatzstück durch zwei Mechanismen auf dem Spritzenkörper gehalten: Zum Einen besteht ein Reibschluss zwischen dem Klemmbereich und dem in radialer Richtung zurückspringenden Bereich des distalen Endes der Spritze. Zum Anderen kann die distale Kante des Klemmbereichs mit der an dem in radialer Richtung zurückspringenden Bereich ausgebildeten Kante zusammenwirken, sodass hier ein Formschluss besteht. Das Zusammenwirken dieser beiden Mechanismen soll verhindern, dass das Aufsatzstück leicht von der Spritze abgezogen werden kann.

Nachteilig an bekannten Spritzen ist, dass die durch den Reibschluss einerseits und den Formschluss andererseits gegebenen Haltekräfte häufig nicht ausreichen, um eine sichere Funktion der Spritze zu gewährleisten. Bei einer Aktivierung oder Betätigung der Spritze werden Kräfte in das Aufsatzstück eingeleitet, die zu einem Lösen der Klemmverbindung und letztendlich zu einer Separation des Aufsatzstücks von der Spritze führen können. Bezüglich des Formschlusses ist insbesondere problematisch, dass in bekannten Systemen die distale Kante des Klemmbereichs einen spitzen Winkel oder einen rechten Winkel aufweist, während die an dem in radialer Richtung zurückspringenden Bereich gebildete Kante eine Fase aufweist beziehungsweise als Rampe ausgebildet ist. Es ergibt sich so zwischen den beiden Kanten eine linienförmige Berührung, wobei keine nennenswerten Reibkräfte aufgebaut werden können. Im Gegenteil ist es möglich, dass die Kante des Klemmbereichs auf der rampenförmigen Kante des zurückspringenden Bereichs abgleitet und sich das Aufsatzstück so relativ leicht von der Spritze abziehen lässt.

Es zeigt sich weiterhin, dass die bekannten Spritzenkörper eine relativ große Längentoleranz aufweisen. Typischerweise wird das Aufsatzstück während der maschinellen Fertigung in einer vorgegebenen Position auf den Spritzenkörper aufgesetzt. Je nach tatsächlicher Länge des individuellen Spritzenkörpers wird das Aufsatzstück dabei in eine Position gebracht, in der es - in axialer Richtung gesehen - näher an dem Spritzenzylinder oder weiter von diesem entfernt angeordnet ist. Da der in radialer Richtung zurückspringende Bereich typischerweise nicht zylindrisch sondern leicht konisch ausgebildet ist, wobei der Außendurchmesser vom distalen Ende auf den Spritzenzylinder hin zunimmt, ergibt sich eine größere Aufdehnung des Klemmbereichs, je näher das Aufsatzstück an dem Spritzenzylinder angeordnet ist. Diese zusätzliche Aufdehnung führt zu einer erhöhten Belastung des Materials und möglicherweise zu einer Überdehnung. Hierdurch können die elastischen Klemmkräfte verringert werden, sodass das Aufsatzstück leichter von der Spritze abgezogen werden kann.

Bekannte Spritzen werden häufig einer Sterilisation unterworfen, nachdem das Aufsatzstück bereits am Spritzenkörper angeordnet ist. Hierbei können Temperaturen erreicht werden, die nahe der Glasübergangstemperatur des Materials liegen, aus dem das Aufsatzstück gebildet ist. In diesem Temperaturbereich kann es im Zuge der Sterilisation zu einer bleibenden Dehnung beziehungsweise Relaxation in dem Material des Aufsatzstücks kommen, sodass wiederum die Klemm- beziehungsweise Haltekräfte reduziert werden und das Aufsatzstück leichter von der Spritze abziehbar ist. Dies ist insbesondere dann problematisch, wenn der Klemmbereich aufgrund einer Anordnung des Aufsatzstücks relativ nahe am Spritzenzylinder bereits stark vorgedehnt ist. Die hierdurch erhöhte Vorbelastung des Materials kann in Zusammenhang mit der Sterilisationstemperatur zu einer Relaxation des Materials führen, sodass die Haltekräfte im besonderen Maße abnehmen.

Aufgabe der Erfindung ist es daher, eine Spritze zu schaffen, die verstärkte Haltekräfte des Aufsatzstücks am Spritzenkörper aufweist, sodass die genannten Nachteile nicht auftreten.

Die Aufgabe wird gelöst durch eine Spritze mit den Merkmalen des Anspruchs 1. Sie zeichnet sich dadurch aus, dass die distale Kante des Klemmbereichs eine Fase aufweist. Diese Fase wirkt mit der an der Kante des in radialer Richtung zurückspringenden Bereichs gebildeten Fase zusammen, sodass hier keine Linienberührung sondern eine Flächenberührung gegeben ist. Auf diese Weise werden in diesem Bereich die Reibungskräfte erhöht, sodass eine größere Haltekraft resultiert. Es muss also eine höhere Kraft aufgewendet werden, um das Aufsatzstück vom Spritzenkörper abzuziehen, als dies bei bekannten Spritzen der Fall ist.

Es wird auch eine Spritze bevorzugt, bei der die Fase an dem zurückspringenden Bereich des distalen Endes der Spritze und die Fase an der distalen Kante des Klemmbereichs des Aufsatzstücks geometrisch aufeinander abgestimmt sind. Hierdurch kann die in diesem Bereich gegebene Flächenreibung optimiert werden, sodass nochmals erhöhte Haltekräfte resultieren.

Besonders bevorzugt wird eine Spritze, bei der die beiden genannten Fasen den gleichen Winkel mit einer Längsachse der Spritze 1 einschließen. So kann sichergestellt werden, dass die Fasen entlang ihrer gesamten Erstreckung aneinander anliegen.

Weitere vorteilhafte Ausgestaltungen ergeben sich aus den Unteransprüchen.

Die Erfindung wird im Folgenden anhand der Zeichnung näher erläutert. Es zeigen:
- Figur 1: eine Schnittdarstellung eines ersten Ausführungsbeispiels einer Spritze, bei der das Aufsatzstück von dem Spritzenzylinder entfernt angeordnet ist;
- Figur 2: eine Ausschnittsvergrößerung aus Figur 1;
- Figur 3: eine weitere Ausschnittsvergrößerung des Ausführungsbeispiels aus Figur 1;
- Figur 4: eine Ausschnittsvergrößerung aus Figur 3;
- Figur 5: ein Ausführungsbeispiel der Spritze, bei dem das Aufsatzstück näher an dem Spritzenzylinder angeordnet ist;
- Figur 6: eine Ausschnittsvergrößerung aus Figur 5, und
- Figuren 7 bis 13: verschiedene Ausführungsbeispiele eines Aufsatzstücks mit verschiedenen Geometrien des Klemmbereichs.

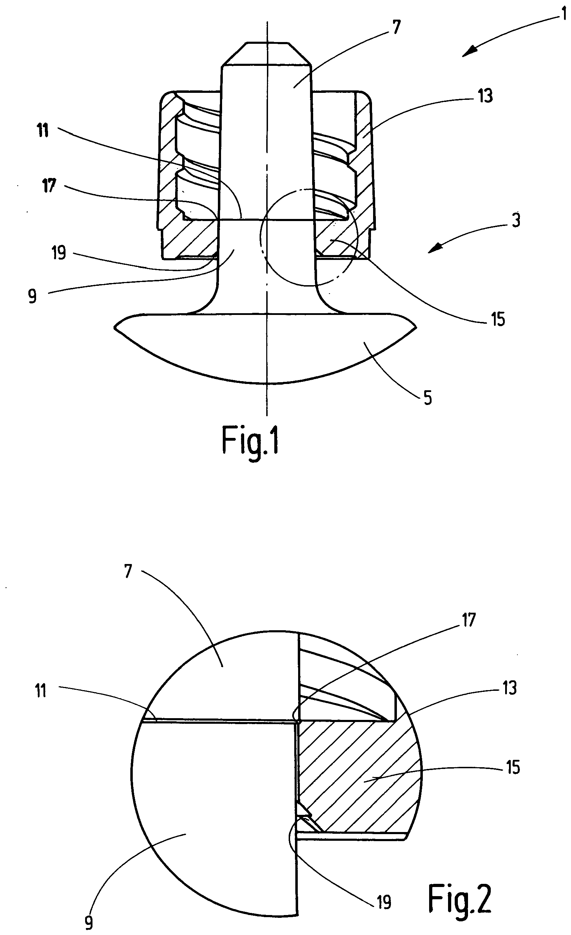

Figur 1 zeigt eine Schnittdarstellung eines ersten Ausführungsbeispiels der Spritze 1. Ein Spritzenkörper 3 ist dabei nur ausschnittsweise zu erkennen. Die Spritze umfasst einen Spritzenzylinder 5, an den sich ein distales Ende 7 anschließt, das als Spritzenkonus ausgebildet ist. Dieses weist einen in radialer Richtung zurückspringenden Bereich 9 auf, der beispielsweise als Rille oder als Nut ausgebildet sein kann. In dem dargestellten Ausführungsbeispiel ist das distale Ende 7 quasi zweigeteilt, sodass ein erster, dem Spritzenzylinder 5 abgewandter Bereich gebildet wird, dessen Durchmesser sich - in Richtung auf den Spritzenzylinder 5 gesehen - vergrößert, sodass dieser Bereich eine konische Außenfläche umfasst, während ein zweiter, dem Spritzenzylinder 5 zugewandter Bereich an der Grenze zwischen den beiden Bereichen einen kleineren Durchmesser als der erste Bereich aufweist und so den zurückspringenden Bereich 9 bildet. Auch dieser zurückspringende Bereich 9 ist in dem dargestellten Ausführungsbeispiel leicht konisch ausgebildet, wobei der Durchmesser - in Richtung auf den Spritzenzylinder 5 gesehen - zunimmt. An seinem dem Spritzenzylinder 5 zugewandten Ende geht der zurückspringende Bereich 9 unmittelbar in den Spritzenzylinder 5 über. Aus diesem Grund vergrößert sich hier der Durchmesser deutlich.

An der Grenze zwischen dem in radialer Richtung zurückspringenden Bereich 9 und dem ersten Bereich wird eine Kante 11 gebildet, an der sich der Durchmesser - in axialer Richtung gesehen - sprunghaft ändert, wobei sich die Kante 11 in Umfangsrichtung um das distale Ende 7 der Spritze 1 erstreckt. Die Kante 11 weist eine in Figur 1 nicht erkennbare Fase auf. Es ist auch möglich, dass die Kante 11 als Rampe ausgebildet ist. Insbesondere wenn der Spritzenkörper 3 aus Glas besteht ist es nicht möglich, eine - im Längsschnitt gesehen - spitzwinklige oder rechtwinklige Kante vorzusehen. Eine solche Kantengeometrie wäre mit zu hohen Spannungen im Material verbunden. Eine Kante 11, die eine Fase aufweist und/oder als Rampe ausgebildet ist, ergibt sich in diesem Fall demnach von selbst aus dem Herstellungsprozess des Spitzenkörpers 3.

Die Spritze 1 umfasst außerdem ein Aufsatzstück 13. Dieses umfasst einen Klemmbereich 15, über den Haltekräfte in den in radialer Richtung zurückspringenden Bereich 9 der Spritze 1 eingeleitet werden. Hierzu weist der Klemmbereich 15 in einem Zustand, in dem das Aufsatzstück 13 von dem Spritzenkörper 3 getrennt vorliegt, einen Innendurchmesser auf, der kleiner ist als der kleinste Außendurchmesser des in radialer Richtung zurückspringenden Bereichs 9. Wird das Aufsatzstück 13 auf den Spritzenkörper aufgesetzt und so positioniert, dass der Klemmbereich 15 mit dem in radialer Richtung zurückspringenden Bereich 9 zusammenwirkt, ergibt sich eine Aufweitung des Klemmbereichs 15, sodass hier elastische Haltekräfte in den in radialer Richtung zurückspringenden Bereich 9 eingeleitet werden.

In dem dargestellten Ausführungsbeispiel ist das Aufsatzstück 13 als Luer Lock ausgebildet. Es dient also zum dichten und sicheren Anschluss von weiteren Injektionselementen an die Spritze 1. In anderen, nicht dargestellten Ausführungsbeispielen kann das Aufsatzstück als Verschlusselement oder als Anschlusselement ausgebildet sein. Wenn das Aufsatzstück 13 als Verschlusselement ausgebildet ist, dient es im Wesentlichen einem dichten und sicheren Verschluss der Spritze 1. Hierbei kann auch eine Garantiefunktion in das Verschlusselement integriert sein. Ist das Aufsatzstück 13 als Anschlusselement ausgebildet, dient es der Kopplung der Spritze 1 mit weiteren Injektionselementen oder als Vial-Adapter beziehungsweise zur Kopplung mit einem Vial-Adapter. Es muss sich hierbei keinesfalls zwingend um eine Kopplung nach Art eines Luer Lock-Verbindungselements handeln. Stattdessen können in verschiedenen Ausführungsbeispielen verschiedene Verbindungselemente beziehungsweise Anschlusselemente zur Anwendung kommen. Wesentlich ist hier lediglich, dass das Aufsatzstück 13 durch einen Klemmbereich 15 auf dem Spritzenkörper 3 gehalten wird.

Der Klemmbereich 15 weist eine distale Kante 17 und eine proximale Kante 19 auf. Bei bekannten Aufsatzstücken ist die distale Kante 17 - im Längsschnitt gesehen - spitzwinklig oder rechtwinklig ausgebildet, sodass bei der dargestellten Position des Aufsatzstücks 13 auf dem Spritzenkörper 3 lediglich eine linienförmige Berührung der distalen Kante 17 mit der gefasten und/oder als Rampe ausgebildeten Kante 11 gegeben sein kann.

Es wird deutlich, dass das Aufsatzstück 13 durch zwei Mechanismen auf dem Spritzenkörper 3 gehalten wird. Zum Einen ergibt sich ein Reibschluss zwischen dem Klemmbereich 15 und dem zurückspringenden Bereich 9, in dem der Klemmbereich 15 gedehnt wird, sodass elastische Haltekräfte in den zurückspringenden Bereich 9 eingeleitet werden. Zum Anderen ergibt sich ein Formschluss, in dem die distale Kante 17 mit der Kante 11 des zurückspringenden Bereichs 9 zusammenwirkt.

Ist zwischen der distalen Kante 17 und der gefasten und/oder als Rampe ausgebildeten Kante 11 nur eine linienförmige Berührung gegeben, wird die Haltekraft an dieser Stelle nicht optimal unterstützt, da nur geringe Reibkräfte vorliegen. Im Gegenteil ist es möglich, dass die Kante 17 über die Fase beziehungsweise über die durch die Kante 11 gebildete Rampe abgleiten kann, wenn Kräfte in axialer Richtung in das Aufsatzstück 13 eingeleitet werden, die geeignet sind, eine Trennung des Aufsatzstücks 13 von dem Spritzenkörper 3 zu bewirken. Solche Kräfte können insbesondere beim Vorbereiten der Spritze 1 zur Injektion, beispielsweise beim Einschrauben von Verbindungselementen in den Luer Lock des dargestellten Ausführungsbeispiels, beim Ausdrücken der Spritze 1 oder auch beim Trennen der Injektionselemente auftreten.

Um die Haltekraft des Aufsatzstücks 13 auf dem Spritzenkörper 3 zu erhöhen, weist die distale Kante 17 des Klemmbereichs 15 eine Fase auf, die in Figur 1 nicht erkennbar ist.

Figur 2 zeigt eine Vergrößerung des Ausschnitts aus Figur 1, der dort mit einem Kreis gekennzeichnet ist. Gleiche und funktionsgleiche Elemente sind mit gleichen Bezugszeichen versehen, sodass auf die vorangegangene Beschreibung verwiesen wird. Hier ist erkennbar, dass die Kante 11 eine Fase aufweist und in dem dargestellten Ausführungsbeispiel insbesondere als Rampe ausgebildet ist. Ebenso ist erkennbar, dass die distale Kante 17 des Klemmbereichs 15 eine Fase aufweist. Auf diese Weise ergibt sich eine Flächenberührung der Fasen der Kanten 11, 17, sodass hier eine erhöhte Reibung gegeben ist, was zur Folge hat, dass insgesamt verbesserte Haltekräfte vorliegen.

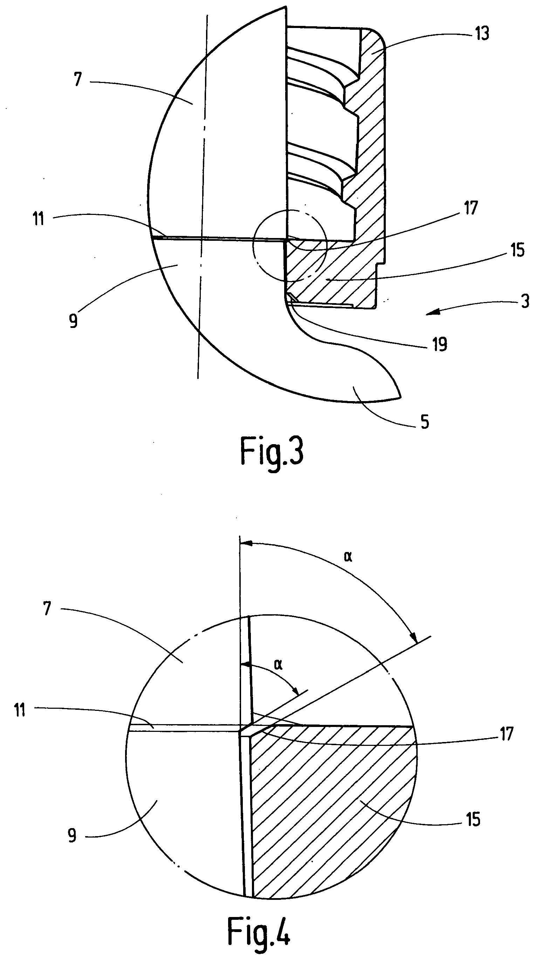

Figur 3 zeigt eine weitere Vergrößerung einer Schnittansicht des Ausführungsbeispiels aus Figur 1. Gleiche und funktionsgleiche Elemente sind mit gleichen Bezugszeichen versehen, sodass auf die vorangegangene Beschreibung verwiesen wird. Hier ist noch deutlicher als in Figur 2 erkennbar, dass sowohl die Kante 11 als auch die Kante 17 eine Fase aufweisen, sodass beide Kanten durch eine flächenförmige Berührung zusammenwirken können.

Figur 4 zeigt eine Vergrößerung des Ausschnitts, der in Figur 3 mit einem Kreis gekennzeichnet ist. Gleiche und funktionsgleiche Elemente sind mit gleichen Bezugszeichen versehen, sodass insofern auf die vorangegangene Beschreibung verwiesen wird. Der Klemmbereich 15 des Aufsatzstücks 13 ist hier in einem gewissen Abstand zum zurückspringenden Bereich 9 des distalen Endes 7 der Spritze 1 gezeichnet, um die hier angesprochenen Merkmale deutlicher hervortreten zu lassen. Um die Reibungsverhältnisse zwischen den gefasten Kanten 11 und 17 weiter zu optimieren, ist in dem dargestellten Ausführungsbeispiel vorgesehen, dass die Kanten geometrisch aufeinander abgestimmt sind. Die Kanten bilden quasi komplementäre Flächen, sodass eine großflächige Berührung und damit eine erhöhte Reibung gegeben ist.

Insbesondere ist in dem dargestellten Ausführungsbeispiel vorgesehen, dass die beiden Fasen der Kante 11 einerseits und der Kante 17 andererseits denselben Winkel α mit einer Längsachse der Spritze 1 einschließen. In diesem Fall ergibt sich eine optimale geometrische Abstimmung der beiden Kanten 11, 17 aufeinander, sodass eine besonders dichte Anlage und damit auch eine besonders hohe Reibung resultiert.

In dem Ausführungsbeispiel, das in den Figuren 1 bis 4 dargestellt ist, ist das Aufsatzstück 13 in einer dem Spritzenzylinder 5 maximal abgewandten Position innerhalb des zurückspringenden Bereichs 9 angeordnet. Dies hat zur Folge, dass die Kanten 11 und 17 unmittelbar aneinander anliegen. Da der zurückspringende Bereich 9 leicht konisch ausgebildet ist, liegt der Klemmbereich 15 diesem hier in einem Bereich kleinsten Durchmessers an, wobei auch die Aufweitung des Klemmbereichs 15 ihren - entlang der Längsachse der Spritze 1 gesehen - minimal möglichen Wert annimmt.

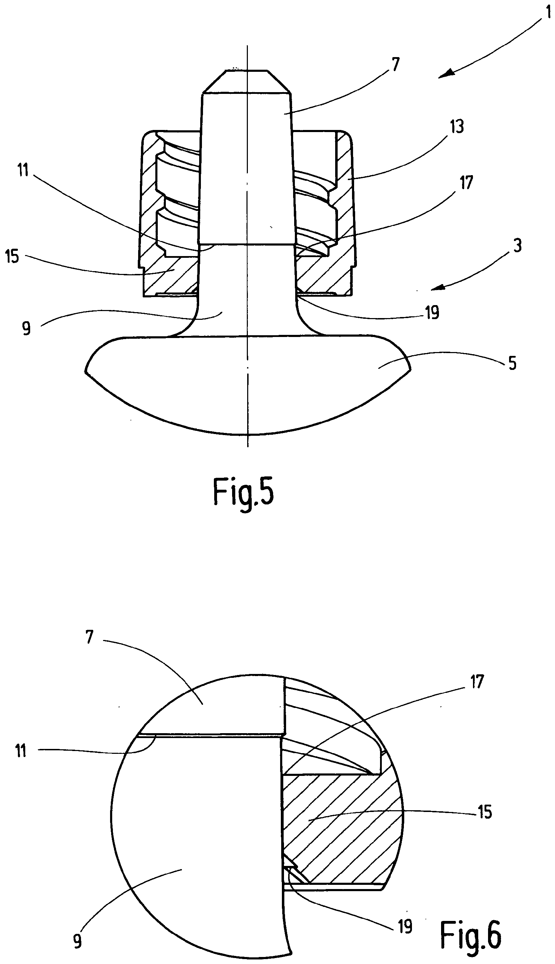

In Figur 5 ist dagegen ein Ausführungsbeispiel dargestellt, in dem das Aufsatzstück 13 in einer Position angeordnet ist, die - entlang der Längsachse der Spritze 1 gesehen - weiter auf den Spritzenzylinder 5 zu verlagert ist. Gleiche und funktionsgleiche Elemente sind mit gleichen Bezugszeichen versehen, sodass insofern auf die vorangegangene Beschreibung verwiesen wird.

Die Anordnung des Aufsatzstücks 13 innerhalb des zurückspringenden Bereichs 9 wird auch als Setzposition bezeichnet. Unterschiedliche Setzpositionen ergeben sich daraus, dass das Aufsatzstück 13 beim Zusammenfügen der Spritze 1 maschinell in eine vorgegebene Position gebracht wird. Der Spritzenkörper 3 weist allerdings bezüglich seiner Gesamtlänge eine Toleranz auf, die beim Aufsetzen des Aufsatzstücks 13 nicht berücksichtigt wird. Demzufolge ergibt sich bei kürzeren Spritzen eine Setzposition des Aufsatzstücks 13, die - in axialer Richtung gesehen - dem Spritzenzylinder 5 weiter abgewandt ist, wie es beispielsweise aus den Figuren 1 bis 4 hervorgeht, während sich bei längeren Spritzen eine sogenannte tiefere Setzposition ergibt, bei der das Aufsatzstück 13 in einer dem Spritzenzylinder 5 - in axialer Richtung gesehen - weiter zugewandten Position angeordnet ist.

Problematisch ist hierbei die Konizität des zurückspringenden Bereichs 9. Da dessen Außendurchmesser in Richtung auf den Spritzenzylinder 5 zunimmt, wird der Klemmbereich 15 in einer tiefen Setzposition stärker aufgeweitet als in einer höheren Setzposition. Dies hat im Fall der tiefen Setzposition erhöhte Materialbelastungen zur Folge. Im ungünstigsten Fall kann der Klemmbereich 15 in einer tiefen Setzposition überdehnt werden, sodass eine dauerhafte Relaxation des Materials resultiert, was zu deutlich reduzierten Reib- und Haltekräften führt.

Insbesondere ist dies dann problematisch, wenn die Spritze 1 mit bereits aufgesetztem Aufsatzstück 13 sterilisiert wird. Hierbei werden typischerweise Temperaturbereiche erreicht, die relativ nah an der Glasübergangstemperatur des Materials liegen, welches das Aufsatzstück 13 umfasst beziehungsweise aus welchem dieses besteht. In diesen Temperaturbereichen kann es zu dauerhaften Veränderungen im Material des Aufsatzstücks 13 kommen, was zu einer Relaxation und einer deutlichen Abnahme der Reib- und Haltekräfte führt.

Um diesen Nachteil zu vermeiden, weist der Klemmbereich des dargestellten Ausführungsbeispiels eine axiale Erstreckung auf, die, bezogen auf die halbe axiale Erstreckung des zurückspringenden Bereichs 9 am distalen Ende 7 der Spritze 1, ungefähr gleich groß ist. Generell wird bevorzugt, dass die axiale Erstreckung des Klemmbereichs 15 verglichen mit der halben axialen Erstreckung des zurückspringenden Bereichs 9 kleiner oder gleich groß ist. Legt man die Gesamtlängentoleranz des Spritzenkörpers 3 zu Grunde, zeigt sich, dass sich in diesem Fall selbst bei der tiefsten anzunehmenden Setzposition der Klemmbereich 15 nicht in einen Bereich hinein erstreckt, der - in axialer Richtung gesehen - so nah am Spritzenzylinder 5 angeordnet ist, dass eine Überdehnung beziehungsweise Relaxation des Klemmbereichs 15 zu befürchten wäre. Die erfindungsgemäß reduzierte axiale Erstreckung des Klemmbereichs 15 führt also dazu, dass unabhängig von der tatsächlichen Länge des Spritzenkörpers 3 innerhalb der Gesamtlängentoleranz effektiv nur noch ein dem Spritzenzylinder 5 abgewandter Bereich des zurückspringenden Bereichs 9 zur Klemmung des Aufsatzstücks 13 verwendet wird. Auf diese Weise wird die Aufweitung des Klemmbereichs 15 auf hinnehmbare Werte beschränkt.

In dem dargestellten Ausführungsbeispiel ist zusätzlich vorgesehen, dass die proximale Kante 19 des Klemmbereichs 15 eine Fase aufweist. Vorzugsweise ist diese Fase relativ breit ausgebildet und trägt damit zu einer weiteren Reduktion der axialen Erstreckung des Bereichs bei, der mit dem zurückspringenden Bereich 9 klemmend zusammenwirkt. Insbesondere bei tiefen Setzpositionen hat die Fase 19 also den Effekt, dass eine unmittelbare Anlage des Klemmbereichs 15 im Bereich größten Durchmessers des zurückspringenden Bereichs 9 vermieden wird. Es wird so nicht nur eine Überdehnung des Klemmbereichs 15 verhindert, sondern gleichzeitig bewirkt, dass der dem Spritzenzylinder 5 abgewandte Bereich des Klemmbereichs sicher und fest an dem zurückspringenden Bereich 9 anliegen kann. Wäre die Kante 19 nicht gefast, würde der Klemmbereich 15 insgesamt auf den ihm an der Kante 19 anliegenden Durchmesser des zurückspringenden Bereichs 9 vorgedehnt. Eine sichere Anlage des dem Spritzenzylinder abgewandten Bereichs wäre aufgrund der Konizität des zurückspringenden Bereichs 9 nicht mehr gegeben.

In dem dargestellten Ausführungsbeispiel weist der Klemmbereich 15 sowohl eine Fase an der proximalen Kante 19 als auch eine reduzierte axiale Erstreckung auf. Es ist auch ein Ausführungsbeispiel möglich, bei dem der Klemmbereich 15 eine reduzierte axiale Erstreckung aufweist, ohne dass die Kante 19 eine Fase aufweist. Ebenso ist ein Ausführungsbeispiel möglich, bei dem der Klemmbereich 15 eine axiale Erstreckung aufweist, die größer ist als die halbe axiale Erstreckung des zurückspringenden Bereichs 9, während die proximale Kante 19 eine Fase aufweist.

Figur 6 zeigt die Vergrößerung des Ausschnitts aus Figur 5, der dort mit einem Kreis gekennzeichnet ist. Gleiche und funktionsgleiche Elemente sind mit gleichen Bezugszeichen versehen, sodass insofern auf die vorangegangene Beschreibung verwiesen wird. Insbesondere ist in Figur 6 deutlich zu erkennen, dass die proximale Kante 19 des Klemmbereichs 15 eine Fase aufweist, wodurch der Bereich effektiver Anlage zwischen dem Klemmbereich 15 und dem zurückspringenden Bereich 9 - in axialer Richtung gesehen - reduziert wird.

Die Haltekräfte des Aufsatzstücks 13 auf dem Spritzenkörper 3 können durch eine vorteilhafte Gestaltung der Geometrie des Klemmbereichs 15 weiter optimiert werden.

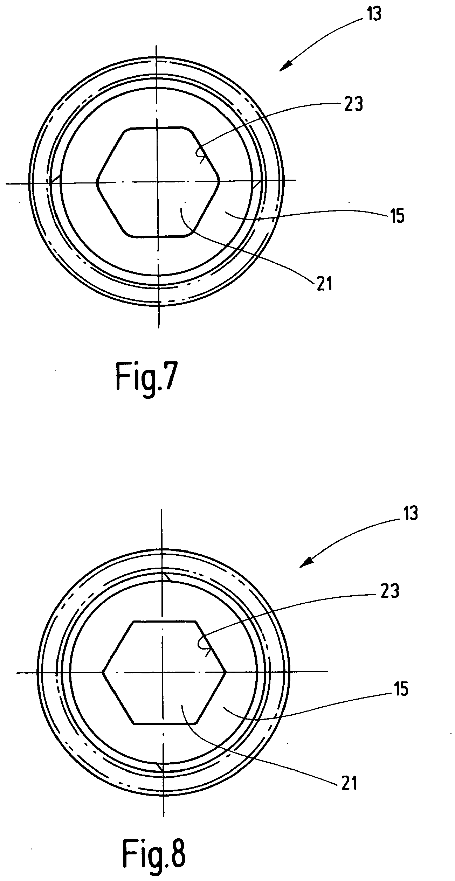

Die Figuren 7 bis 13 zeigen jeweils eine Ansicht von verschiedenen Ausführungsbeispielen eines Aufsatzstücks 13, die orthogonal zu der Ansicht orientiert ist, die in den Figuren 1 bis 6 dargestellt ist. Die Blickrichtung ist hierbei entlang der Längsachse des Aufsatzstücks 13 gerichtet. Gleiche und funktionsgleiche Elemente sind mit gleichen Bezugszeichen versehen, sodass insofern auf die vorangegangene Beschreibung verwiesen wird. Aus Figur 7 geht hervor, dass der Klemmbereich 15 eine zentrale Ausnehmung 21 aufweist, die in zusammengesetztem Zustand den zurückspringenden Bereich 9 des distalen Endes 7 der Spritze 1 aufnimmt. Die Ausnehmung 21 hat in dem in Figur 7 dargestellten Ausführungsbeispiel eine hexagonale Geometrie, wodurch sechs äquivalente Anlageflächen gebildet werden, von denen beispielhaft eine mit der Bezugsziffer 23 bezeichnet ist. Der Klemmbereich 15 ist hier also - in Draufsicht gesehen - hexagonal ausgebildet. Es zeigt sich, dass eine hexagonale Form des Klemmbereichs bezüglich der in den radial zurückspringenden Bereich 9 eingeleiteten Haltekräfte besonders vorteilhaft ist. Insbesondere ermöglicht die hexagonale Geometrie des Klemmbereichs 15 aufgrund der erhöhten Haltekräfte eine Reduzierung der axialen Erstreckung des Klemmbereichs 15, sodass diese verglichen mit der halben axialen Erstreckung des zurückspringenden Bereichs 9 kleiner oder gleich groß sein kann, ohne dass sich der hierdurch bedingte Verlust an Reibungsfläche negativ auf die Haltekräfte auswirken würde.

In dem in Figur 7 dargestellten Ausführungsbeispiel sind die Ecken, an denen zwei äquivalente Anlageflächen 23 aneinandergrenzen, abgerundet.

Figur 8 zeigt ein Ausführungsbeispiel, das dem den in Figur 7 dargestellten sehr ähnlich ist. Ein Unterschied besteht darin, dass die Ecken von aufeinanderstoßenden Anlageflächen 23 nicht abgerundet sind. Jedenfalls ergibt sich auch so ein hexagonaler Klemmbereich 15, der - in Umfangsrichtung gesehen - durchgängig ausgebildet ist und sechs äquivalente Anlageflächen 23 aufweist.

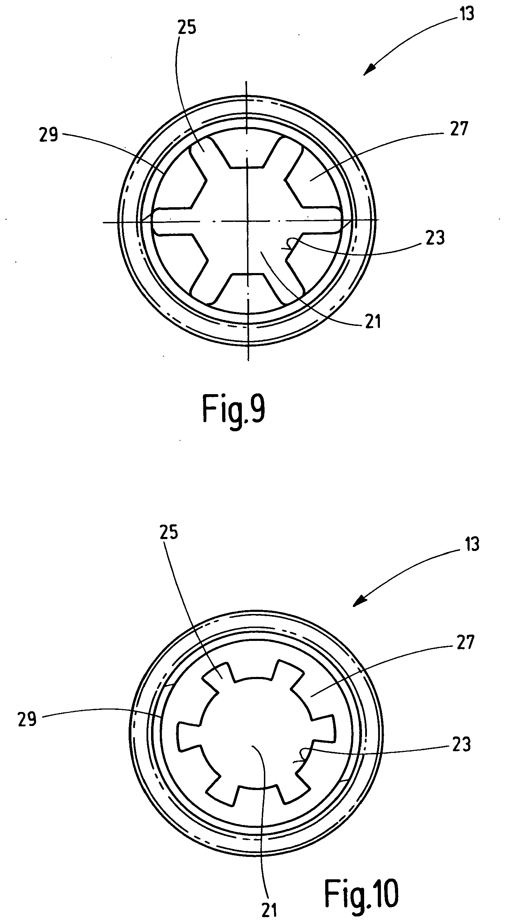

Der Klemmbereich 15 des in Figur 9 dargestellten Ausführungsbeispiels ist dagegen - in Umfangsrichtung gesehen - nicht durchgehend ausgebildet sondern weist mindestens eine Ausnehmung auf, sodass mindestens eine Klemmbacke gebildet wird. Das konkret gezeigte Ausführungsbeispiel weist - in Umfangsrichtung gesehen - sechs äquivalente Ausnehmungen 25 auf, die im Wesentlichen radial verlaufen, sodass auch sechs äquivalente Klemmbacken 27 gebildet werden. Die Klemmbacken 27 umgeben die Ausnehmung 21, die in zusammengesetztem Zustand den zurückspringenden Bereich 9 des distalen Endes 7 der Spritze 1 aufnimmt. Jede der Klemmbacken 27 weist eine äquivalente Anlagefläche 23 auf, über die Haltekräfte in den in radialer Richtung zurückspringenden Bereich 9 der Spritze 1 eingeleitet werden. Die mindestens eine Ausnehmung 25 hat in Figur 9 eine in radialer Richtung langgestreckte Form und erstreckt sich von der Ausnehmung 21 bis zu einem äußeren Rand 29 des Klemmbereichs 15.

Das Ausführungsbeispiel gemäß Figur 10 weist ebenfalls mindestens eine Ausnehmung 25, hier konkret sechs äquivalente Ausnehmungen 25 auf. Es werden also auch hier sechs äquivalente Klemmbacken 27 gebildet. Im Unterschied zu dem Ausführungsbeispiel aus Figur 9 erstrecken sich allerdings die Ausnehmungen 25 von der Ausnehmung 21 in radialer Richtung nicht bis zum äußeren Rand 29 des Klemmbereichs 15, sondern nur etwa bis zur Hälfte dieser Erstreckung. Der Klemmbereich 15 umfasst dadurch einen - in Umfangsrichtung gesehen - zusammenhängenden Bereich, der - in radialer Richtung gesehen - dem äußeren Rand 29 zugewandt ist. Indem die radiale Erstreckung der mindestens einen Ausnehmung 25 variiert wird, kann die Federelastizität der einzelnen Klemmbacken 27 eingestellt werden. Erstrecken sich die Ausnehmungen 25 - wie in Figur 9 gezeigt - über die gesamte radiale Ausdehnung des Klemmbereichs 15, weisen die Klemmbacken 27 eine hohe Federelastizität. Je weniger weit sich die Ausnehmungen 25 von der Ausnehmung 21 in Richtung auf den äußeren Rand 29 des Klemmbereichs 15 erstrecken, desto geringer ist die Federelastizität der einzelnen Klemmbacken 27 auf. Auf diese Weise kann über eine Variation der radialen Erstreckung der Ausnehmungen 25 auch eine Veränderung der in den zurückspringenden Bereich 9 eingeleiteten Haltekräfte bewirkt werden. Die Haltekräfte können auch durch die Breite der Ausnehmungen 25 vorbestimmt werden: Je breiter die Ausnehmungen sind um so mehr nehmen die Haltekräfte ab. Auch die Kontur der Ausnehmungen, deren Breite hier von innen nach außen zunimmt, kann zur Einstellung der Haltekräfte speziell gewählt werden: Je breiter die Ausnehmungen radial außen sind, umso geringer sind die Haltekräfte.

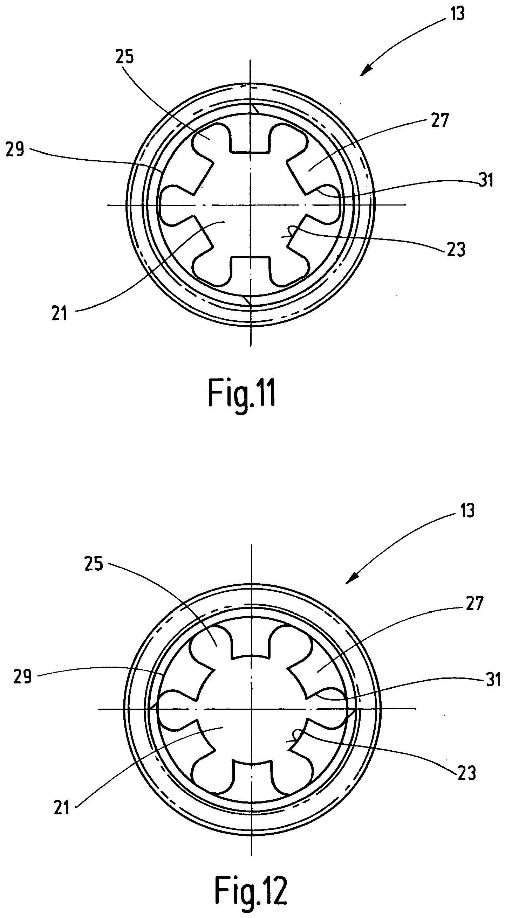

In Figur 11 weisen die Ausnehmungen 25 - in Draufsicht gesehen - quasi eine Tropfenform auf. Hierdurch weisen die Klemmbacken 27 Kanten 31 auf, die senkrecht auf den Anlageflächen 23 stehen. Dies führt im äußeren Bereich des Klemmbereichs 15 zu einem - in Umfangsrichtung gesehen - vergrößerten Abstand der einzelnen Klemmbacken 27 voneinander. Die radiale Erstreckung der Ausnehmungen 25 reicht auch in diesem Ausführungsbeispiel nicht vollständig bis zum äußeren Rand 29. Die Federelastizität der Klemmbacken 27 kann durch ein Zusammenspiel der in Draufsicht gesehenen Form der Ausnehmung 25 und deren radialer Erstreckung variiert werden. Sind die Ausnehmungen 25 - wie hier dargestellt - beispielsweise tropfenförmig ausgebildet, wobei sich ein - in Umfangsrichtung gesehen - vergrößerter Abstand der einzelnen Klemmbacken 27 voneinander ergibt, führt dies zu einer erhöhten Federelastizität der Klemmbacken 27. Form und radiale Erstreckung der Ausnehmungen 25 können also aufeinander abgestimmt werden, um eine gewünschte Federelastizität der Klemmbacken 27 und damit einen gewünschten Wert der Haltekräfte, die in den zurückspringenden Bereich 9 eingeleitet werden, zu erzielen.

Auch in Figur 12 sind die Ausnehmungen 25 - in Draufsicht gesehen - tropfenförmig ausgebildet. Ihre radialer Erstreckung reicht allerdings von der Ausnehmung 21 bis zum äußeren Rand 29 des Klemmbereichs 15. Im Unterschied zu den vorangegangenen Ausführungsbeispielen sind hier allerdings die Anlageflächen 23 nicht plan, sondern gekrümmt ausgebildet, sodass sich Zylinderabschnittsflächen ergeben. Auf diese Weise ist die Ausnehmung 21 - in Draufsicht gesehen - nicht durch ein Hexagon begrenzt, sondern durch einen Kreis. Bevorzugt folgt die Krümmung der zylinderabschnittsflächenförmigen Anlageflächen 23 der Krümmung des zurückspringenden Bereichs 9, sodass in dem dargestellten Ausführungsbeispiel eine besonders großflächige Berührung zwischen den Anlageflächen 23 und dem zurückspringenden Bereich 9 gegeben ist. Dies führt wiederum zu erhöhten Haltekräften.

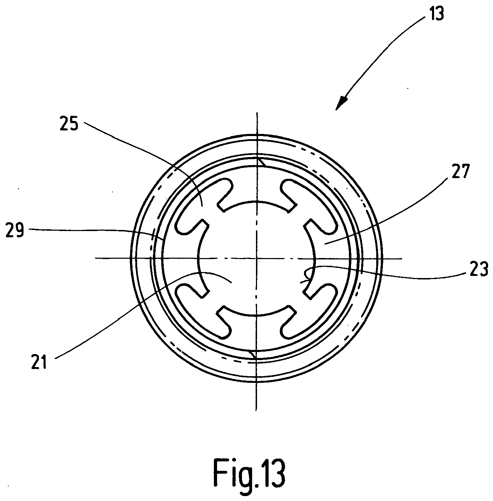

Figur 13 zeigt ein Ausführungsbeispiel einer Klemmbereichsgeometrie, die lediglich vier Ausnehmungen 25 aufweist. Die Ausnehmungen 25 haben eine - in Umfangsrichtung gesehen - langgestreckte, ovale Form, wobei sie symmetrisch zur kurzen Achse des Ovals angeordnete Durchbrechungen in Richtung auf die Ausnehmung 21 umfassen, sodass vier Klemmbacken 27 gebildet werden. Die Anlageflächen 23 sind wiederum zylinderabschnittsflächenförmig gekrümmt, sodass die Ausnehmung 21 - in Draufsicht gesehen - von einem Kreis begrenzt wird. Die - in Umfangsrichtung gesehen - langgestreckte, ovale Form der Ausnehmungen 25 hat in Zusammenhang mit den Durchbrechungen zur Folge, dass Hinterschneidungen hinter den Klemmbacken 27 gebildet werden, was deren Elastizität erhöht. Gleichzeitig können die Anlageflächen 23 auf diese Weise relativ groß ausfallen, sodass ein erheblicher Teil der zur Verfügung stehenden Zylinderfläche zur Einleitung von Haltekräften in den zurückspringenden Bereich 9 genutzt wird. In radialer Richtung erstrecken sich die Ausnehmungen 25 auch in diesem Ausführungsbeispiel nicht vollständig von der Ausnehmung 21 bis zum äußeren Rand 29. Wesentlich an dem dargestellten Ausführungsbeispiel ist allerdings, dass durch die spezielle, in Umgangsrichtung langgestreckte Geometrie der Ausnehmungen 25 eine hohe Elastizität der Klemmbacken 27 bei gleichzeitig großen Anlageflächen 23 möglich ist.

Selbstverständlich kann jede der in den Figuren 7 bis 13 dargestellten Klemmbereichsgeometrien beliebig mit den übrigen in Zusammenhang mit den Figuren 1 bis 6 beschriebenen Merkmalen kombiniert werden, um eine erfindungsgemäße Spritze 1 zu erhalten.

Insgesamt zeigt sich, dass die vorliegende Erfindung vergrößerte Haltekräfte zwischen einem Aufsatzstück 13 und einem Spritzenkörper 3 bereitstellt, sodass diese Elemente insbesondere bei der Vorbreitung einer Injektion nicht mehr versehentlich voneinander getrennt werden können. Gleichzeitig vermeidet die vorliegende Erfindung den Nachteil, dass eine tiefe Setzposition des Aufsatzstücks 13 auf dem Spritzenkörper 3 zu einer Belastung oder Überdehnung des Klemmbereichs 15 unter Verlust von Haltekräften führen kann. Ebenso wird der Nachteil vermieden, dass es bei einer Sterilisation des mit dem Spritzenkörper 3 verbundenen Aufsatzstücks 13 zu einer Relaxation im Material desselben unter gleichzeitigem Verlust von Haltekräften kommen kann.

## Patentansprüche

1. Spritze mit
- einem Spritzenzylinder (5),
- einem distalen Ende (7),
- das als Spritzenkonus ausgebildet ist, wobei
- das distale Ende (7) einen in radialer Richtung zurückspringenden Bereich (9) aufweist,
- sodass eine sich in Umfangsrichtung erstreckende Kante (11) gebildet wird, wobei
- die Kante (11) eine Fase aufweist, und mit
- einem Aufsatzstück (13), das
• einen Klemmbereich (15) umfasst, wobei
• von dem Aufsatzstück (13) über den Klemmbereich (15) Haltekräfte in den in radialer Richtung zurückspringenden Bereich (9) der Spritze (1) eingeleitet werden, und wobei
• der Klemmbereich (15) eine distale Kante (17) umfasst,
**dadurch gekennzeichnet, dass**
- die distale Kante (17) des Klemmbereichs (15) eine Fase aufweist, und dass
- die Fase an dem zurückspringenden Bereich (9) des distalen Endes (7) der Spritze (1) und die Fase an der distalen Kante (17) des Klemmbereichs (15) des Aufsatzstücks (13) geometrisch aufeinander so abgestimmt sind, dass sich eine Flächenberührung ergibt.

2. Spritze nach Anspruch 1 **dadurch gekennzeichnet, dass** die beiden Fasen denselben Winkel (α) mit einer Längsachse der Spritze (1) einschließen.

3. Spritze nach einem der vorhergehenden Ansprüche, **dadurch gekennzeichnet, dass** der Klemmbereich (15) eine proximale Kante (19) umfasst, die eine Fase aufweist.

4. Spritze nach einem der vorhergehenden Ansprüche, **dadurch gekennzeichnet, dass** der Klemmbereich (15) - in Umfangsrichtung gesehen - mindestens eine Ausnehmung (25) aufweist, sodass mindestens eine Klemmbacke (27) gebildet wird.

5. Spritze nach einem der vorhergehenden Ansprüche, **dadurch gekennzeichnet, dass** der Klemmbereich (15) - in Draufsicht gesehen - hexagonal ausgebildet ist.

6. Spritze nach einem der vorhergehenden Ansprüche, **dadurch gekennzeichnet, dass** der Klemmbereich (15) eine axiale Erstreckung aufweist, die, bezogen auf die halbe axiale Erstreckung des zurückspringenden Bereichs (9) am distalen Ende (7) der Spritze (1), kleiner oder gleich groß ist.

7. Spritze nach einem der vorhergehenden Ansprüche, **dadurch gekennzeichnet, dass** der zurückspringende Bereich (9) als Rille oder als Nut ausgebildet ist.

8. Spritze nach einem der vorhergehenden Ansprüche, **dadurch gekennzeichnet, dass** das Aufsatzstück (13) als Verschlusselement ausgebildet ist.

9. Spritze nach einem der vorhergehenden Ansprüche, **dadurch gekennzeichnet, dass** das Aufsatzstück (13) als Anschlusselement ausgebildet ist.

10. Spritze nach einem der vorhergehenden Ansprüche, **dadurch gekennzeichnet, dass** das Aufsatzstück (13) als Luer Lock ausgebildet ist.

## Claims

1. Syringe with
- a syringe cylinder (5),
- a distal end (7)
- that is configured as a syringe cone, wherein
- the distal end (7) comprises a region (9) that is set back in radial direction,
- thereby forming an edge (11) that extends in the circumferential direction, wherein
- the edge (11) comprises a chamfer, and with
- an attachment piece (13)
• comprising a clamping region (15), wherein
• holding forces are introduced from the attachment piece (13) via the clamping region (15) into the region (9) set back in radial direction of the syringe (1), and wherein
• the clamping region (15) comprises a distal edge (17),
**characterized in that**
- the distal edge (17) of the clamping region (15) comprises a chamfer, and that
- the chamfer at the set-back region (9) of the distal end (7) of the syringe (1) and the chamfer at the distal end (17) of the clamping region (15) of the attachment piece (13) are geometrically harmonized with each other such that a surface area contact is caused.

2. Syringe according to claim 1, **characterized in that** the two chamfers enclose the same angle (α) with a longitudinal axis of the syringe (1).

3. Syringe according to one of the preceding claims, **characterized in that** the clamping region (15) comprises a proximal edge (19) having a chamfer.

4. Syringe according to one of the preceding claims, **characterized in that** the clamping region (15) - seen in circumferential direction - comprises at least one recess (25) resulting in the formation of at least one clamping jaw (27).

5. Syringe according to one of the preceding claims, **characterized in that** the clamping region (15) - seen in a top view - is configured in a hexagonal shape.

6. Syringe according to one of the preceding claims, **characterized in that** the clamping region (15) has an axial extension that is, in relation to half the axial extension of the set-back region (9), smaller or of equal size at the distal end (7) of the syringe (1).

7. Syringe according to one of the preceding claims, **characterized in that** the set-back region (9) is configured as a groove or a slot.

8. Syringe according to one of the preceding claims, **characterized in that** the attachment piece (13) is configured as a closure element.

9. Syringe according to one of the preceding claims, **characterized in that** the attachment piece (13) is configured as a connection element.

10. Syringe according to one of the preceding claims, **characterized in that** the attachment piece (13) is configured as a Luer lock.

## Revendications

1. Seringue avec
- un cylindre de seringue (5),
- une extrémité distale (7),
- qui est réalisée comme cône de seringue, dans laquelle
- l'extrémité distale (7) présente une région (9) reculant en direction radiale,
- de sorte qu'une arête (11) s'étendant dans la direction périphérique est formée, dans laquelle
- l'arête (11) présente un chanfrein, et avec
- une pièce de chapeau (13) qui
• comprend une région de serrage (15), dans laquelle
• des forces de maintien sont introduites dans la région (9) de la seringue (1) reculant en direction radiale par la pièce de chapeau (13) par le biais de la région de serrage (15), et dans laquelle
• la région de serrage (15) comprend une arête distale (17),
**caractérisée en ce que**
- l'arête distale (17) de la région de serrage (15) présente un chanfrein, et que
- le chanfrein sur la région reculant (9) de l'extrémité distale (7) de la seringue (1) et le chanfrein sur l'arête distale (17) de la région de serrage (15) de la pièce de chapeau (13) sont adaptés l'un à l'autre géométriquement de sorte qu'il en résulte un contact superficiel.

2. Seringue selon la revendication 1, **caractérisée en ce que** les deux chanfreins incluent le même angle (α) avec un axe longitudinal de la seringue (1).

3. Seringue selon une des revendications précédentes, **caractérisée en ce que** la région de serrage (15) comprend une arête proximale (19) qui présente un chanfrein.

4. Seringue selon une des revendications précédentes, **caractérisée en ce que** la région de serrage (15) présente au moins un évidement (25), vue dans la direction périphérique, de sorte qu'au moins une mâchoire de serrage (27) est formée.

5. Seringue selon une des revendications précédentes, **caractérisée en ce que** la région de serrage (15) est réalisée de manière hexagonale, vue en vue de dessus.

6. Seringue selon une des revendications précédentes, **caractérisée en ce que** la région de serrage (15) présente une étendue axiale qui est inférieure ou égale par rapport à la demie étendue axiale de la région reculant (9) à l'extrémité distale (7) de la seringue (1).

7. Seringue selon une des revendications précédentes, **caractérisée en ce que** la région reculant (9) est réalisée comme cannelure ou comme rainure.

8. Seringue selon une des revendications précédentes, **caractérisée en ce que** la pièce de chapeau (13) est réalisée comme élément de fermeture.

9. Seringue selon une des revendications précédentes, **caractérisée en ce que** la pièce de chapeau (13) est réalisée comme élément de raccord.

10. Seringue selon une des revendications précédentes, **caractérisée en ce que** la pièce de chapeau (13) est réalisée comme Luer Lock.
